# EUROPEAN PATENT SPECIFICATION

(11) **EP 1 159 285 B1**
(45) Date of publication and mention of the grant of the patent: **25.05.2005**
(21) Application number: 00914914.7
(22) Date of filing: 07.03.2000
(51) Int. Cl.: C07H 21/04, C12P 19/34

(54) **METHODS AND COMPOSITIONS FOR ECONOMICALLY SYNTHESIZING AND ASSEMBLING LONG DNA SEQUENCES**
SYNTHESEVERFAHREN ZUM ÖKONOMISCHEN AUFBAU LANGER DNA-SEQUENZEN UND ZUSAMMENSETZUNGEN HIERFÜR
TECHNIQUES ET COMPOSITIONS PERMETTANT D'EFFECTUER LA SYNTHESE ET L'ASSEMBLAGE DE SEQUENCES D'ADN LONGUES DE MANIERE ECONOMIQUE

(30) Priority: 08.03.1999 US 264388
(43) Date of publication of application: 05.12.2001
(73) Proprietor: Metrigen, Inc., Burlingame, CA 94010 (US)
(72) Inventor: BRENNAN, Thomas, M., San Francisco, CA 94109 (US); HEYNEKER, Herbert, L., San Francisco, CA 94115 (US)
(74) Representative: Schüssler, Andrea, Dr.
(86) International application number: PCT/US2000/006335
(87) International publication number: WO 2000/053617

(56) References cited:
- EP-A- 0 359 545
- WO-A-94/27719
- DE-A- 19 736 591
- US-A- 5 641 648
- STEMMER, WILLEM P. C. ET AL: "Single-step assembly of a gene and entire plasmid from large numbers of oligodeoxyribonucleotides" GENE (1995), 164(1), 49-53 ,1995, XP004041916

## Description

### FIELD OF THE INVENTION

The present invention relates to a cost-effective method of assembling long DNA sequences from short synthetic oligonucleotides. More specifically, short oligonucleotides are synthesized *in situ* on a solid support and subsequently cleaved from the solid support prior to or during the assembly into the full-length sequences.

### BACKGROUND OF THE INVENTION

The advent of rapid sequencing technology has created large databases of DNA sequences containing useful genetic information. The remaining challenges are to find out what these gene products really do, how they interact to regulate the whole organism, and ultimately how they may be manipulated to find utility in gene therapy, protein therapy, and diagnosis. The elucidation of the function of genes requires not only the knowledge of the wild type sequences, but also the availability of sequences containing designed variations in order to further the understanding of the roles various genes play in health and diseases. Mutagenesis is routinely conducted in the laboratory to create random or directed libraries of interesting sequence variations. However the ability to manipulate large segments of DNA to perform experiments on the functional effects of changes in DNA sequences has been limited by the availability of modified enzymes and their associated costs. For example, the researcher cannot easily control the specific addition or deletion of certain regions or sequences of DNA via traditional mutagenesis methods, and must resort to the selection of interesting DNA sequences from libraries containing genetic variations.

It would be most useful if a researcher could systematically synthesize large regions of DNA to determine the effect of differences in sequences upon the function of such regions. However, DNA synthesis using traditional methods is impractical because of the declining overall yield. For example, even with a yield of 99.5% per step in the phosphoramidite method of DNA synthesis, the total yield of a full length sequence of 500 base pairs long would be less than 1%. Similarly, if one were to synthesize overlapping strands of, for example, an adenovirus useful as a gene therapy vector, the 50-70 kilobases of synthetic DNA required, even at a recent low price of approximately $1.00 per base, would cost over $50,000 per full sequence, far too expensive to be practical.

The recovery of long segments of DNA may be improved when the DNA chemical synthesis is combined with recombinant DNA technology. Goeddel *et al*., *Proc. Natl. Acad. Sci. USA* 76(1):106-110 (1979); Itakura *et al., Science* 198:1056-1063 (1977); and Heyneker *et al., Nature* 263:748-752 (1976). The synthesis of a long segment of DNA may begin with the synthesis of several modest-sized DNA fragments by chemical synthesis and continue with enzymatic ligation of the modest-sized fragments to produce the desired long segment of DNA. Synthetically made modest-sized DNA fragments may also be fused to DNA plasmids using restriction enzymes and ligase to obtain the desired long DNA sequences, which may be transcribed and translated in a suitable host. Recently, self-priming PCR technology has been used to assemble large segments of DNA sequences from a pool of overlapping oligonucleotides by using DNA polymerase without the use of ligase. *Dillon et al., BioTechniques* 9(3):298-300 (1990); Prodromou *et al., Protein Engineering* 5(8):827-829 (1992); Chen *et al., J. Am. Chem. Soc*. 116:8799-8800 (1994); and Hayashi *et al., BioTechniques* 17(2):310-315 (1994). Most recently, DNA shuffling method was introduced to assemble genes from random fragments generated by partial DNAaseI digestion or from a mixture of oligonucleotides. Stemmer *et al, Nature* 370:389-391 (1994); Stemmer *et al, Proc. Natl. Acad. Sci. USA* 91:10747-10751 (1994); Stemmer *et al, Gene* 164:49-53 (1996); Crameri *et al., Nat. Biotechnol*. 15:436-438 (1997); Zhang *et al., Proc. Natl*. *Acad. Sci. USA* 94:4504-4509 (1997); Crameri *et al., Nature* 391:288-291 (1998); Christians *et al., Nat. Biotechnol.* 17:259-264 (1999), U.S. Patent Nos. 5,830,721, 5,811,238, 5,830,721, 5,605,793, 5,834,252, and 5,837,458; and PCT publications WO 98/13487, WO98/27230, WO 98/31837, WO 99/41402, 99/57128, and WO 99/65927.

Methods for synthesizing a large variety of short or modest-sized oligonucleotides have been extensively described. One of the methods is to use microarray technology, where a large number of oligonucleotides are synthesized simultaneously on the surface of a solid support. The microarray technology has been described in *Green et al., Curr. Opin. in Chem. Biol*. 2:404-410 (1998), Gerhold *et al*., *TIBS*, 24:168-173 (1999), U.S. Patent Nos. 5,510,270, 5,412,087, 5,445,934, 5,474,796, 5,744,305, 5,807,522, 5,843,655, 5,985,551, and 5,927,547. One method for synthesizing high density arrays of DNA fragments on glass substrates uses light-directed combinatorial synthesis. However, the photolithographic synthesis method provides oligonucleotides which are neither pure enough for later enzymatic assembly nor a method which is flexible and cost effective. For example, due to the low chemical coupling yield of *in situ* synthesis using photolithography, each oligonucleotide may contain a substantial number of truncated products in addition to the desired length oligonucleotides. For example, in 10-mers and 20-mers, only about 40% and 15% of the oligonucleotides are of the full length respectively. Forman, J., *et al*., Molecular Modeling of Nucleic Acids, Chapter 13, pp 206-228, American Chemical Society (1998)) and McGall *et al., J. Am. Chem. Soc.,* 119:5081-5090 (1997). In addition, several thousands of dollars of masks specific to any given series of sequences are required for practical assembly.

Existing methods for the synthesis of long DNA sequences also have many drawbacks, for example, the length limitations of conventional solid phase DNA synthesis, the requirement of synthesizing both strands of DNA, and the complexity of multiple enzymatic reactions for stepwise assembly. These drawbacks inevitably add to the cost of obtaining long DNA sequences. There is a need in the art to economically synthesize multiple oligonucleotides and subsequently assemble them into long DNA sequences. Such an inexpensive and custom synthesis and assembly process has many uses. Gene sequences of interest can be assembled and tested for a variety of functionalities, for example, the function of relative position of promoter to gene coding sequence, the role of introns versus exons, the minimization of gene sequence necessary for function, the role of polymorphisms and mutations, the effectiveness of sequence changes to gene therapy vectors, the optimization of the gene coding for a protein for a specific experiment or industrial application, among others. These functional analysis may be explored with the DNA designs truly under the control of the researcher. In other cases, specific variations in assembled sequence can be used to create structured libraries containing many possible genetic variations for testing of the function or the inhibition of the function. Eventually entire genomes could be easily synthesized, assembled, and functionally tested in this manner. In short, any experiment in which a model system of synthetic genes or genomes could be changed in a specific way under the control of a researcher, could be performed easily and less expensively.

### SUMMARY OF THE INVENTION

According to the present invention there is provided a method for producing a DNA sequence of greater than 200 bases long comprising the steps of:
(a) synthesizing on a functionalized solid support at least ten oligonucleotides from about 10 to 200 bases encoding for either the sense or antisense strand of said DNA sequence wherein said oligonucleotides are covalently attached to the solid support using a cleavable moiety;
(b) cleaving said oligonucleotides from the solid support; and
(c) assembling the oligonucleotides into said DNA sequence.

The present method for synthesizing and assembling long DNA sequences from short oligonucleotides comprises the steps of: (a) synthesizing on a solid support an array of oligonucleotide sequences wherein the oligonucleotides collectively encode both strands of the target DNA and are covalently attached to the solid support using a cleavable moiety; (b) cleaving the oligonucleotides from the solid support; and (c) assembling the oligonucleotides into the target full-length sequence. The target long DNA sequences contemplated in the present invention may be a regulatory sequence, a gene or a fragment thereof, a vector, a plasmid, a virus, a full genome of an organism, or any other biologically functional DNA sequences which may be assembled from overlapping oligonucleotides, either directly or indirectly by enzymatic ligation, by using a DNA polymerase, by using a restriction enzyme, or by other suitable assembly methods known in the art.

In preferred embodiments, oligonucleotides may be prepared by *in situ* synthesis on a solid support. In particular, the *in situ* synthesis of oligonucleotides may employ the "drop-on-demand" method, which uses technology analogous to that employed in ink-jet printers. In addition, hydrophilic/hydrophobic arrays or surface tension arrays, which consist of patterned regions of hydrophilic and hydrophobic surfaces, may be employed. Preferably, the size of the long DNA sequence ranges from about 200 to 10,000 bases, oligonucleotide may be in the range of about 10 to 200 bases long, more preferably, in the range of about 20 to 100 bases long. Preferably, the number of oligonucleotides synthesized on the solid support is from about 10 to 200, more preferably, from about 10 to 500.

### BRIEF DESCRIPTION OF THE FIGURES

Figure 1 shows hydroxyl-group bearing non-cleavable linkers used for hybridisation directly on the glass chip.
Figure 2 shows the coupling of a chemical phosphorylation agent as the special amidite to allow cleavage of the oligonucleotide after synthesis.
Figure 3 shows the amidite (TOPS) used to prepare universal CPG-support to allow cleavage of the oligonucleotide after synthesis.
Figure 4A illustrate the formation of an array surface that is ready for solid phase synthesis.
Figure 4B illustrates O-Nitrocarbamate array making chemistry.
Figure 5 illustrates surface tension wall effect at the dot-interstice interface. The droplet containing solid phase synthesis reagents does not spread beyond the perimeter of the dot due to the surface tension wall.
Figure 6 illustrates hydrogen-phosphonate solid phase oligonucleotide synthesis on an array surface.
Figure 7A illustrates the top view of a piezoelectric impulse jet of the type used to deliver solid phase synthesis reagents to individual dots in the array plate synthesis methods.
Figure 7B illustrates the side view of a piezoelectric impulse jet of the type used to deliver solid phase synthesis reagents to individual dots in the array plate synthesis methods.
Figure 8 illustrates use of a piezoelectric impulse jet head to deliver blocked nucleotides and activating agents to individual dots on an array plate. The configuration shown has a stationary head/moving plate assembly.
Figure 9 illustrates an enclosure for array reactions showing array plate, sliding cover and manifolds for reagent inlet and outlet.
Figure 10 illustrates the gene assembly process from short synthetic oligonucleotides.

### DETAILED DESCRIPTION OF THE INVENTION

The present invention relates to a cost-effective method for assembling long DNA sequences from short synthetic oligonucleotides. In general, the present method for synthesizing and assembling long DNA sequences from synthetic oligonucleotides comprises the steps of: (a) synthesizing on a solid support an array of oligonucleotide sequences wherein the oligonucleotides collectively encode both strands of the target DNA and are covalently attached to the solid support using a cleavable moiety; (b) cleaving the oligonucleotides from the solid support; and (c) assembling the oligonucleotides into the target full-length sequence. The target long DNA sequences contemplated in the present invention may be a regulatory sequence, a gene or a fragment thereof, a vector, a plasmid, a virus, a full genome of an organism, or any other DNA sequences which may be assembled from overlapping oligonucleotides, either directly or indirectly by enzymatic ligation, by using a DNA polymerase, by using a restriction enzyme, or by other suitable assembly methods known in the art.

### Attachment of a cleavable moiety to the oligonucleotides and the solid support

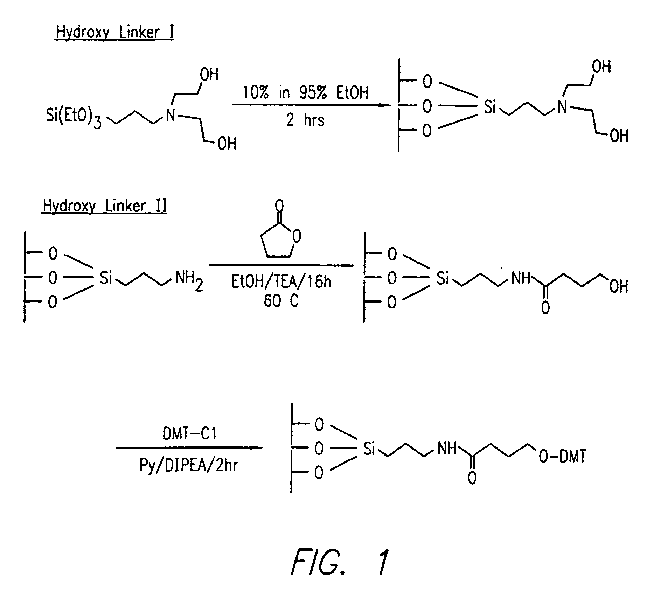

In solid phase or microarray oligonucleotide synthesis designed for diagnostic and other hybridization-based analysis, the final oligonucleotide products remain attached to the solid support such as controlled-pore glass (CPG) or chips. A non-cleavable linker such as the hydroxyl linker I or II in Figure 1 is typically used. These hydroxyl linkers remain intact during the deprotection and purification processes and during the hybridization analysis. Synthesis of a large number of overlapping oligonucleotide for the eventual assembly into a longer DNA segment, however, is performed on a linker which allows the cleavage of the synthesized oligonucleotide. The cleavable moiety is removed under conditions which do not degrade the oligonucleotides. Preferably the linker may be cleaved using two approaches, either (a) simultaneously under the same conditions as the deprotection step or (b) subsequently utilizing a different condition or reagent for linker cleavage after the completion of the deprotection step. The former approach may be advantageous, as the cleavage of the linker is performed at the same time as the deprotection of the nucleoside bases. Time and effort are saved to avoid additional post-synthesis chemistry. The cost is lowered by using the same reagents for deprotection in the linker cleavage. The second approach may be desirable, as the subsequent linker cleavage may serve as a pre-purification step, eliminating all protecting groups from the solution prior to assembly.

Any suitable solid supports may be used in the present invention. These materials include glass, silicon, wafer, polystyrene, polyethylene, polypropylene, polytetrafluorethylene, among others. Typically, the solid supports are functionalized to provide cleavable linkers for covalent attachment to the oligonucleotides. The linker moiety may be of six or more atoms in length. Alternatively, the cleavable moiety may be within an oligonucleotide and may be introduced during *in situ* synthesis. A broad variety of cleavable moieties are available in the art of solid phase and microarray oligonucleotide synthesis. Pon, R., "Solid-Phase Supports for Oligonucleotide Synthesis" in "Protocols for oligonucleotides and analogs; synthesis and properties," *Methods Mol. Biol.* 20:465-496 (1993); Verma *et al*., *Annu. Rev. Biochem.* 67:99-134 (1998); and U.S. Patent Nos. 5,739,386, 5,700,642 and 5,830,655. A suitable cleavable moiety may be selected to be compatible with the nature of the protecting group of the nucleoside bases, the choice of solid support, the mode of reagent delivery, among others. The cleavage methods may include a variety of enzymatic, or non-enzymatic means, such as chemical, thermal, or photolytic cleavage. Preferably, the oligonucleotides cleaved from the solid support contain a free 3'-OH end. The free 3'-OH end may also be obtained by chemical or enzymatic treatment, following the cleavage of oligonucleotides.

The covalent immobilization site may either be at the 5' end of the oligonucleotide or at the 3'end of the oligonucleotide. In some instances, the immobilization site may be within the oligonucleotide (*i.e.* at a site other than the 5' or 3' end of the oligonucleotide). The cleavable site may be located along the oligonucleotide backbone, for example, a modified 3'-5' internucleotide linkage in place of one of the phosphodiester groups, such as ribose, dialkoxysilane, phosphorothioate, and phosphoramidate internucleotide linkage. The cleavable oligonucleotide analogs may also include a substituent on or replacement of one of the bases or sugars, such as 7-deazaguanosine, 5-methylcytosine, inosine, uridine, and the like.

In one embodiment, cleavable sites contained within the modified oligonucleotide may include chemically cleavable groups, such as dialkoxysilane, 3'-(S)-phosphorothioate, 5'-(S)-phosphorothioate, 3'-(N)-phosphoramidate, 5'-(N)-phosphoramidate, and ribose. Synthesis and cleavage conditions of chemically cleavable oligonucleotides are described in U.S. Patent Nos. 5,700,642 and 5,830,655. For example, depending upon the choice of cleavable site to be introduced, either a functionalized nucleoside or a modified nucleoside dimer may be first prepared, and then selectively introduced into a growing oligonucleotide fragment during the course of oligonucleotide synthesis. Selective cleavage of the dialkoxysilane may be effected by treatment with fluoride ion. Phosphorothioate internucleotide linkage may be selectively cleaved under mild oxidative conditions. Selective cleavage of the phosphoramidate bond may be carried out under mild acid conditions, such as 80% acetic acid. Selective cleavage of ribose may be carried out by treatment with dilute ammonium hydroxide.

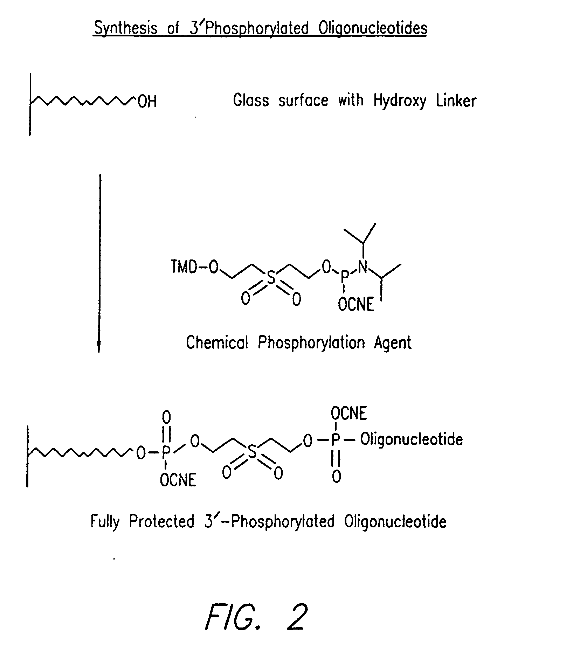

In preferred embodiments, in order to convert the non-cleavable hydroxyl linker (Figure 1) into a cleavable linker, a special phosphoramidite may be coupled to the hydroxyl group prior to the phophoramidite or H-phosphonate oligonucleotide synthesis. One preferred embodiment of such special phophoramidite, a chemical phosphorylation agent, is shown in Figure 2. The reaction conditions for coupling the hydroxyl group with the chemical phosphorylation agent are known to those skilled in the art. The cleavage of the chemical phosphorylation agent at the completion of the oligonucleotide synthesis yields an oligonucleotide bearing a phosphate group at the 3' end. The 3'-phosphate end may be converted to a 3' hydroxyl end by a treatment with a chemical or an enzyme, such as alkaline phosphatase, which is routinely carried out by those skilled in the art.

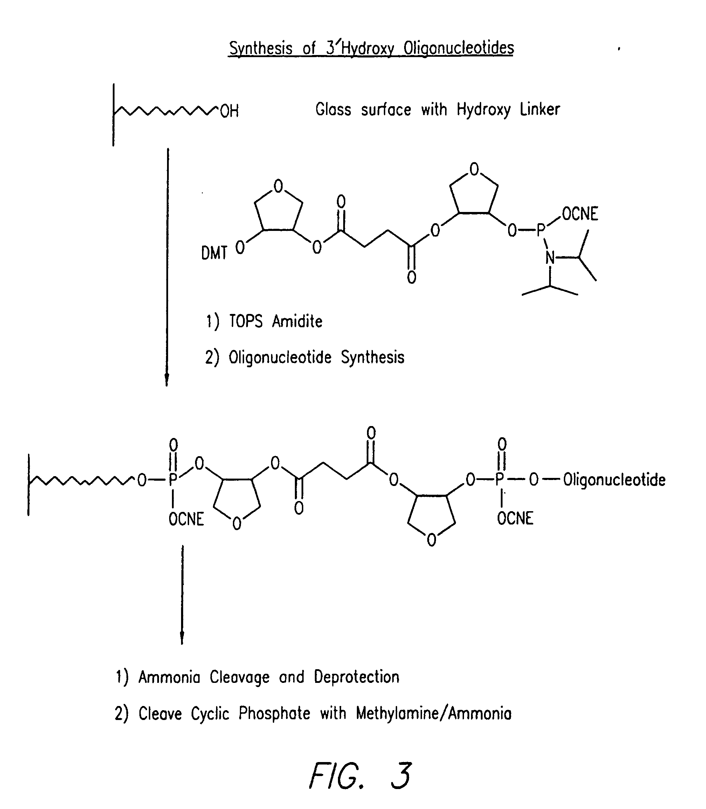

Another class of cleavable linkers is described by McLean, *et al*. in PCT publication WO 93/20092. This class of cleavable linker, also known as TOPS for two oligonucleotides per synthesis, was designed for generating two oligonucleotides per synthesis by first synthesizing an oligonucleotide on a solid support, attaching the cleavable TOPS linker to the first oligonucleotide, synthesizing a second oligonucleotide on the TOPS linker, and finally cleaving the linker from both the first and second oligonucleotides. In the present invention however, the TOPS phosphoramidite may be used to convert a non-cleavable hydroxyl group on the solid support to a cleavable linker, suitable for synthesizing a large number of overlapping oligonucleotides. A preferred embodiment of TOPS reagents is the Universal TOPS™ phosphoramidite, which is shown in Figure 3. The conditions for Universal TOPS™ phosphoramidite preparation, coupling and cleavage are detailed in Hardy *et al., Nucleic Acids Research* 22(15):2998-3004 (1994), which is incorporated herein by reference. The Universal TOPS™ phosphoramidite yields a cyclic 3' phosphate that may be removed under basic conditions, such as the extended amonia and/or ammonia/methylamine treatment, resulting in the natural 3' hydroxy oligonucleotide.

A cleavable amino linker may also be employed in the synthesis of overlapping oligonucleotides. The resulting oligonucleotides bound to the linker via a phosphoramidite linkage may be cleaved with 80% acetic acid yielding a 3'-phosphorylated oligonucleotide.

In another embodiment, cleavable sites contained within the modified oligonucleotide may include nucleotides cleavable by an enzyme such as nucleases, glycosylases, among others. A wide range of oligonucleotide bases, e.g. uracil, may be removed by DNA glycosylases, which cleaves the N-glycosylic bond between the base and deoxyribose, thus leaving an abasic site. Krokan *et. al., Biochem. J*. 325:1-16 (1997). The abasic site in an oligonucleotide may then be cleaved by Endonuclease IV, leaving a free 3'-OH end. In another embodiment, the cleavable site may be a restriction endonuclease cleavable site, such as class IIs restriction enzymes. For example, BpmI, BsgI, BseRI, BsmFI, and FokI recognition sequence may be incorporated in the immobilized oligonucleotides and subsequently cleaved to release oligonucleotides.

In another embodiment, the cleavable site within an immobilized oligonucleotide may include a photocleavable linker, such as ortho-nitrobenzyl class of photocleavable linkers. Synthesis and cleavage conditions of photolabile oligonucleotides on solid support are described in Venkatesan *et al. J. of Org. Chem*. 61:525-529 (1996), Kahl et al., *J. of Org. Chem*. 64:507-510(1999), Kahl *et al., J. of Org. Chem.* 63:4870-4871 (1998), Greenberg *et al., J. of Org. Chem*. 59:746-753 (1994), Holmes *et al*., *J. of Org*. *Chem*. 62:2370-2380 (1997), and U.S. Patent No. 5,739,386. Ortho-nitobenzyl-based linkers, such as hydroxymethyl, hydroxyethyl, and Fmoc-aminoethyl carboxylic acid linkers, may also be obtained commercially.

### Determination of overlapping oligonucleotides encoding the long DNA sequence of interest

The present invention represents a general method for synthesizing and assembling any long DNA sequence from an array of overlapping oligonucleotides. Preferably, the size of the long DNA region ranges from about 200 to 10,000 bases. More preferably, the size of the long DNA region ranges from about 400 to 5,000 bases. The long DNA sequence of interest may be split into a series of overlapping oligonucleotides. With the enzymatic assembly of the long DNA sequence, it is not necessary that every base, both the sense and antisense strand, of the long DNA sequence of interest be synthesized. The overlapping oligonucleotides are typically required to collectively encode both strands of the DNA region of interest. The length of each overlapping oligonucleotide and the extent of the overlap may vary depending on the methods and conditions of oligonucleotide synthesis and assembly. Several general factors may be considered, for example, the costs and errors associated with synthesizing modest size oligonucleotides, the annealing temperature and ionic strength of the overlapping oligonucleotides, the formation of unique overlaps and the minimization of non-specific binding and intramolecular base pairing, among others. Although, in principle, there is no inherent limitation to the number of overlapping oligonucleotides that may be employed to assemble them in to the target sequence, the number of overlapping oligonucleotides is preferably from about 10 to 2000, and more preferably, from about 10 to 500.

In particular, for the assembly method using a DNA polymerase, a unique overlap is preferred in order to produce the correct size of long DNA sequence after assembly. Unique overlaps may be achieved by increasing the degree of overlap. However, increasing the degree of overlap adds the number of bases required, which naturally incurs additional cost in oligonucleotide synthesis. Those skilled in the art will select the optimal length of the overlapping oligonucleotides and the optimal length of the overlap suitable for oligonucleotide synthesis and assembly methods. In particular, a computer search of both strands of the target sequence with the sequences of each of the overlap regions may be used to show unique design of oligonucleotides with the least likelihood of give nonspecific binding. Preferably, the length of each oligonucleotide may be in the range of about 10 to 200 bases long. More preferably, the length of each oligonucleotide is in the range of about 20 to 100 bases long. Preferably, oligonucleotides overlap their complements by about 10 to 100 bases. The lowest end of the range, at least a 10-base overlap, is necessary to create stable priming of the polymerase extension of each strand. At the upper end, maximally overlapped oligonucleotides of 200 bases long would contain 100 bases of complementary overlap. Most preferably, the overlapping regions, in the range of about 15-20 base pairs in length may be designed to give a desired melting temperature, e.g., in the range of 52-56 °C, to ensure oligonucleotide specificity. It may also be preferred that all overlapping oligonucleotides have a similar extent of overlap and thus a similar annealing temperature, which will normalize the annealing conditions during PCR cycles.

### Oligonucleotide synthesis

Synthesis of oligonucleotides may be best accomplished using a variety of chip or microarray based oligonucleotide synthesis methods. Traditional solid phase oligonucleotide synthesis on controlled-pore glass may be employed, in particular when the number of oligonucleotides required to assemble the desired DNA sequence is small. Oligonucleotides may be synthesized on an automated DNA synthesizer, for example, on an Applied Biosystems 380A synthesizer using 5-dimethoxytritylnucleoside β-cyanoethyl phosphoramidites. Synthesis may be carried out on a 0.2 µM scale CPG solid support with an average pore size of 1000 Å. Oligonucleotides may be purified by gel electrophoresis, HPLC, or other suitable methods known in the art.

In preferred embodiments, oligonucleotides may be prepared by *in situ* synthesis on a solid support in a step-wise fashion. With each round of synthesis, nucleotide building blocks may be added to growing chains until the desired sequence and length are achieved in each spot. In particular, the *in situ* synthesis of oligonucleotides may employ the "drop-on-demand" method, which uses technology analogous to that employed in ink-jet printers. U.S. Patent Nos. 5,474,796, 5,985,551, 5,927,547, Blanchard *et al., Biosensors and Bioelectronics* 11:687-690 (1996), and Schena *et al., TIBTECH* 16:301-306 (1998). This approach typically utilizes piezoelectric or other forms of propulsion to transfer reagents from miniature nozzles to solid surfaces. For example, the printer head travels across the array, and at each spot, electric field contracts, forcing a microdroplet of reagents onto the array surface. Following washing and deprotection, the next cycle of oligonucleotide synthesis is carried out. The step yields in piezoelectric printing method typically equal to, and even exceed, traditional CPG oligonucleotide synthesis. The drop-on-demand technology allows high-density gridding of virtually any reagents of interest. It is also easier using this method to take advantage of the extensive chemistries already developed for oligonucleotide synthesis, for example, flexibility in sequence designs, synthesis of oligonucleotide analogs, synthesis in the 5'-3' direction, among others. Because ink jet technology does not require direct surface contact, piezoelectric delivery is amendable to very high throughput.

In preferred embodiments, a piezoelectric pump may be used to add reagents to the *in situ* synthesis of oligonucleotides. Microdroplets of 50 picoliters to 2 microliters of reagents may be delivered to the array surface. The design, construction, and mechanism of a piezoelectric pump are described in U.S. Patent Nos. 4,747,796 and 5,985,551. The piezoelectric pump may deliver minute droplets of liquid to a surface in a very precise manner. For example, a picopump is capable of producing picoliters of reagents at up to 10,000 Hz and accurately hits a 250 micron target at a distance of 2 cm.

In preferred embodiments of the instant invention, hydrophilic/hydrophobic arrays or surface tension arrays, which consist of patterned regions of hydrophilic and hydrophobic surfaces, may be employed. U.S. Patent Nos. 4,747,796 and 5,985,551. A hydrophilic/hydrophobic array may contain large numbers of hydrophilic regions against a hydrophobic background. Each hydrophilic region is spatially segregated from neighboring hydrophilic region because of the hydrophobic matrix between hydrophilic spots. Surface tension arrays described in may be employed in the present invention. Typically the support surface has about 10-50 x 10⁻¹⁵ moles of functional binding sites per mm² and each functionalized binding site is about 50-2000 microns in diameter.

There are significant advantages to making arrays by surface tension localization and reagent microdelivery. The lithography and chemistry used to pattern the substrate surface are generic processes that simply define the array feature size and distribution. They are completely independent from the oligonucleotide sequences that are synthesized or delivered at each site. In addition, the oligonucleotide synthesis chemistry uses standard rather than custom synthesis reagents. The combined result is complete design flexibility both with respect to the sequences and lengths of oligonucleotides used in the array, the number and arrangement of array features, and the chemistry used to make them. There is no cost associated with changing the composition of an array once it has been designed. This method provides an inexpensive, flexible, and reproducible method for array fabrication.

Essentially, the fabrication of hydrophilic/hydrophobic arrays may involve coating a solid surface with a photoresist substance and then using a generic photomask to define the array patterns by exposing them to light. Positive or negative photoresist substances are well known to those of skill in the art. For example, an optical positive photoresist substance, for example, AZ 1350 (Novolac™ type-Hoechst Celanese™, Novolac™ is a proprietary novolak resin, which is the reaction product of phenols with formaldehyde in an acid condensation medium), or an E-beam positive photoresist substance, for example, EB-9 (polymethacrylate by Hoya™) may be used.

The photoresist substance coated surface is subsequently exposed and developed to create a patterned region of a first exposed support surface. The exposed surface is then reacted with a fluoroalkylsilane to form a stable hydrophobic matrix. The remaining photoresist is then removed and the glass chip reacted with an amonisilane or hydroxy group bearing silane to form hydrophilic spots. Alternatively, the patterned support surface may be made by reacting a support surface with a hydroxy or aminoalkylsilane to form a derivatized hydrophilic support surface. The support surface is then reacted with o-nitrobenzyl carbonyl chloride as a temporary photolabile blocking to provide a photoblocked support surface. The photoblocked support surface is then exposed to light through a mask to create unblocked areas on the support surface with unblocked hydroxy or aminoalkylsilane. The exposed surface is then reacted with perfluoroalkanoyl halide or perfluoroalkylsulfonyl halide to form a stable hydrophobic (perfluoroacyl or perfluoroalkylsulfonamido) alkyl siloxane matrix. This remaining photoblocked support surface is finally exposed to create patterned regions of the unblocked hydroxy or aminoalkylsilane to form the derivatized hydrophilic binding site regions. A number of siloxane functionalizing reagents may be used. For example, hydroxyalkyl siloxanes, diol (dihydroxyalkyl) siloxanes, aminoalkyl siloxanes, and dimeric secondary aminoalkyl siloxanes, may be used to derive the patterned hydrophilic hydroxyl or amino regions.

A number of solid supports may be used in oligonucleotide synthesis using a piezoelectric pump. There are two important characteristics of the masked surfaces in patterned oligonucleotide synthesis. First, the masked surface must be inert to the conditions of ordinary oligonucleotide synthesis. The masked surface must present no free hydroxy or amino groups to the bulk solvent interface. Second, the surface must be poorly wet by common organic solvents such as acetonitrile and the glycol ethers, relative to the more polar functionalized binding sites. The wetting phenomenon is a measure of the surface tension or attractive forces between molecules at a solid-liquid interface, and is defined in dynes/cm². Fluorocarbons have very low surface tension because of the unique polarity (electronegativity) of the carbon-flourine bond. In tightly structured Langmuir-Blodgett type films, surface tension of a layer is primarily determined by the percent of fluorine in the terminus of the alkyl chains. For tightly ordered films, a single terminal trifluoromethyl group will render a surface nearly as lipophobic as a perfluoroalkyl layer. When fluorocarbons are covalently attached to an underlying derivatized solid (highly crosslinked polymeric) support, the density of reactive sites will generally be lower than Langmuir-Blodgett and group density. However, the use of perfluoroalkyl masking agents preserves a relatively high fluorine content in the solvent accessible region of the supporting surface.

Glass (polytetrasiloxane) are particularly suitable for patterned oligonucleotide synthesis using a piezoelectric pump to deliver reagents, because of the numerous techniques developed by the semiconductor industry using thick films (1-5 microns) of photoresists to generate masked patterns of exposed glass surfaces. The first exposed glass surface may be derivatized preferably with volatile fluoroalkyl silanes using gas phase diffusion to create closely packed lipophobic monolayers. The polymerized photoresist provides an effectively impermeable barrier to the gaseous fluoroalkyl silane during the time period of derivatization of the exposed region. Following lipophobic derivatization however, the remaining photoresist can be readily removed by dissolution in warm, organic solvents (methyl, isobutyl, ketone, or N-methyl pyrrolidone) to expose a second surface of raw glass, while leaving the first applied silane layer intact. This second region glass may then be derivatized by either solution or gas phase methods with a second, polar silane which contains either a hydroxyl or amino group suitable for anchoring solid phase oligonucleotide synthesis. Siloxanes have somewhat limited stability under strongly alkaline conditions.

A number of organic polymers also have desirable characteristics for patterned hydrophilic/hydrophobic arrays. For example, Teflon (polytetrafluoroethylene) may provide an ideal lipophobic surface. Patterned derivatization of this type of material may be accomplished by reactive ion or plasma etching through a physical mask or using an electron beam, followed by reduction to surface hydroxymethyl groups. Polypropylene/polyethylene may be surface derivatized by gamma irradiation or chromic acid oxidation, and converted to hydroxy or aminomethylated surfaces. Highly crosslinked polystryene-divinylbenzene (ca. 50%) is non-swellable, and may be readily surface derivatized by chloromethlylation and subsequently converted to other functional groups. Nylon provides an initial surface of hexylamino groups, which are directly active. The lipophobic patterning of these surfaces may be effected using the same type of solution-based thin film masking techniques and gas phase derivatization as glass, or by direct photochemical patterning using o-nitrobenzylcarbonyl blocking groups. Subsequent to the patterning of these surfaces, suitable cleavable linkers are coupled to the reactive group such as the hydroxy or amino group before the addition of nucleoside phosphoramidite.

After derivatizing the initial silane as described above, assembly of oligonucleotides on the prepared dots is carried out according to the phosphoramidite method. Acetonitrile is replaced by a high-boiling mixture of adiponitrile and acetonitrile (4:1) in order to prevent evaporation of the solvent on an open glass surface. Delivery of the blocked phosphoramidites and the activator (S-ethyl-tetrazole) is directed to individual spots using a picopump apparatus. All other steps including detritylation, capping, oxidation and washing, are performed on the array in a batch process by flooding the surface with the appropriate reagents.

Upon the completion of synthesis, the overlapping oligonucleotides may be cleaved prior to or during the assembly step. For example, the oligonucleotides may be cleaved from solid supports by standard deprotection procedures, such as ammonia or methylamine/ammonia treatment. The resulting mixture of deprotected oligonucleotides may be directly used for assembly.

### Assembly of overlapping oligonucleotides into the full-length target sequence

Assembly of the target long DNA sequence from a series of overlapping oligonucleotides may be accomplished using a variety of methods in the literature known to those skilled in the art. The standard approach is to use enzymatic ligation to arrive at the target DNA of the desired length. The overlapping oligonucleotides may be annealed to form double-strand DNA sequence with single stranded and/or double-stranded breaks. These breaks may then be filled in with a DNA polymerase and/or enzymatically ligated using DNA ligases using known methods in the art. Intermolecular ligation of the 5' and 3' ends of oligonucleotides through the formation of a phosphodiester bond typically requires one oligonucleotide bearing a 5'-phosporyl donor group and another with a free 3'-hydroxyl acceptor. Oligonucleotides may be phosphorylated using known methods in the art. The full-length target DNA sequence may then be amplified using PCR-based methods or cloned into a vector of choice using known methods in the art. Additional assembly methods also include the use of a restriction enzyme or a DNA polymerase.

### 1. Assembly using a restriction enzyme

Restriction enzymes may be employed to assemble short oligonucleotides into long DNA sequences. A restriction enzyme is a group of enzymes that cleave DNA internally at specific base sequences. As an example, oligonucleotide directed double-strand break repair may be used. Mandecki, *Proc. Natl. Acad. Sci. USA* 83:7177-7181 (1986); Mandecki *et al*., *Gene* 68:101-107 (1988) and Mandecki *et al., Gene* 94:103-107 (1990). In general, this method comprises three steps: (1) cloning suitable inserts in a plasmid DNA; (2) generating restriction fragments with protruding ends from the insert containing plasmid DNA; and (3) assembling the restriction fragments into the target long DNA sequence.

In preferred embodiments, cloning of inserts in a plasmid DNA in step 1 may be carried out using oligonucleotide directed double-strand break repair, also known as the bridge mutagenesis protocol. The oligonucleotide directed double-strand break repair essentially involves the transformation of *E. coli* with a denatured mixture of one or more oligonucleotide inserts and a linearized plasmid DNA, wherein the 5' and 3' ends of the oligonucleotide inserts are homologous to sequences flanking the double-strand break (i.e., the cleavage site) of the linearized plasmid DNA. The homologous sequences at the 5' and 3' ends of the oligonucleotide inserts direct, *in vivo*, the repair of the double-strand break of the linearized plasmid DNA. The homologous sequence between each side of the double-strand break and each end of the oligonucleotide insert is typically more than 10 nt long. In preferred embodiments, the homologous sequences at the 5' and 3' end of oligonucleotides and the two sides of the double-strand break contain *Fok*I recognition sites (5'-GGATG-3'). A series of overlapping subsequences of the target DNA sequence are inserted between two *Fok*I sites.

The *Fok*I restriction enzyme creates a staggered double-strand break at a DNA site 9 and 13 nt away from its recognition site, which upon cleavage of the plasmid DNA with *Fok*I, a restriction is liberated that contains unique 4 nt long 5' protruding ends. The uniqueness of ends permits efficient and direct simultaneous ligation of the restriction fragments to form the target long DNA sequence. The oligonucleotide inserts using the *Fok*I method of gene assembly are designed by dividing the target long DNA sequence into a series of subsequences of clonable size, typically in the range of 20 nt to 200 nt. The division points may be preferably between the codons of the open reading frame (ORF) of the target long DNA sequence. Each subsequence may overlap its neighboring subsequence on either side by four nt, so that the overlapping regions will form complementary cohesive ends when the cloned subsequences are removed from the plasmid DNA with *Fok*I restriction enzyme. In particular, the overlapping subsequences are typically chosen such that they are unique, which will assure that they may be annealed to each other in the proper arrangement during the assembly of subsequences into the target long DNA sequence following the *Fok*I cleavage. In particular, if there is any *Fok*I site within the target DNA sequence, it may be preferably placed within an overlap region, which causes *Fok*I cleavage at this site to fall outside the cloned regions. Once the subsequences containing the four nt overlap have been determined, sequences of the oligonucleotide inserts may be obtained by adding two arms to provide the necessary sequence homology to two sides of the double-strand break of the DNA plasmid. The sequence of oligonucleotide inserts thus take the form of arm1 + subsequence (containing 4 nt overlap) + arm2, in which arm1 and arm2, each containing the *Fok*I site, are homologous to the respective side of the double-strand break of the DNA plasmid. The total length of the oligonucleotide inserts may be varied to optimize the efficiency of break repair. In addition, position of the subsequence with respect to the homologous sites may also be varied to optimize the efficiency of repair. It is known that the efficiency of repair decreases as the distance between the subsequence and the homologous sequence increases. In particularly preferred embodiments using the *Fok*I method of gene assembly, the average length of a subsequence is about 70 nt and arm1 and arm2 of the oligonucleotide inserts are 15 nt each, containing the *Fok*I site and sequences complementary to sequences flanking the double-strand break of the plasmid DNA. Therefore, in particularly preferred embodiments, the average length of the oligonucleotide insert is 100 nt (arm 1 + subsequence + arm2).

The oligonucleotide inserts may then be cloned into a suitable vector by the bridge mutagenesis protocol. A suitable plasmid system may be selected based on the existence of a cluster of unique restriction sites for cleaving plasmid DNA and the feasibility of an easy and accurate screening method for the insert containing colonies of plasmid. A color screening method is particularly preferred. The pUC plasmid, which contains multiple cloning sites and an indicator gene (the *lac*Z gene or a fragment thereof) may be used. In particular, a frame shift mutation may be introduced to the multiple cloning site of pUC in order to effect a suitable screening method. For example, a deletion of one residue at the *Pst*I site may be introduced to the pUC plasmid. The oligonucleotide insert introduced into the mutated plasmid may then contain one extra nucleotide to restore the reading frame of the *lac*Z when the repair of the double-strand break of the plasmid DNA occurs. This way, the insert containing plasmids are readily selected, as the repaired plasmid (or the insert containing) form blue colonies, while the cells containing the nucleotide deletion (the parent) plasmid gives rise to white colonies. It is also advantageous that all insertions introduced into plasmid are designed such that they would destroy a unique restriction site within the multiple cloning sites and at the same time create a new restriction site. This feature would allow for an additional confirmation of the insertion event by restriction digestion of plasmid DNA. Suitable DNA plasmid used for the *Fok*I method of gene assembly may be cut with a restriction enzyme, preferably a unique restriction site. While it is convenient that the linearized plasmid is obtained by restriction enzyme cleavage at one site, the present invention also contemplates other methods for generating the linearized plasmid DNA, such as, by restriction enzyme cleavage at multiple sites, reconstructing a linear plasmid by ligating DNA fragments, or random cleavage of DNA using DNase digestion, sonication, among others. The linearized DNA plasmid may be mixed with the oligonucleotide inserts under denaturing conditions and the mixture may be transformed into a suitable organism, such as *E. coli* with suitable compotent cells using known methods in the art. Conditions may be varied to improve the efficiency of repair, for example, the molar ratio of oligonucleotide inserts and the plasmid DNA, the denaturing conditions, among others. Typically, a molar excess of oligonucleotide over plasmid DNA is necessary for efficient repair of the double-strand, typically in the range from 10-fold to 1000-fold molar excess of oligonucleotide inserts. It may also be necessary to denature the linear plasmid DNA before using transformation, for example by incubating the mixture of plasmid NDA and oligonucleotide inserts at 100°C for 3 min. Plasmid constructs containing the *Fok*I fragments may be selected using the designed screening method.

The insert containing DNA plasmid may then be digested with *Fok*I (New England BioLabs) under the conditions recommended by the manufacture. The *Fok*I fragments may then be purified and joined together in a single ligation reaction according to a standard protocol known in the art. The *Fok*I fragments of the oligonucleotide inserts contain subsequences with unique complementary 4-bp overhangs which, when annealed and ligated, formed the target long DNA sequence. It should be noted that the ligation of *Fok*I restriction fragment is not limited to DNA fragments introduced by the bridge mutagenesis. Protruding ends with 4 nt 5' overhangs may be generated by other methods, for example, by *Fok*I digestion of any DNA sequence. Successful assembly of the target long DNA sequence may be verified by DNA sequencing, hybridization-based diagnostic method, molecular biology techniques, such as restriction digest, selection marker, or other suitable methods. DNA manipulations and enzyme treatments are carried out in accordance with established protocols in the art and manufacturers' recommended procedures. Suitable techniques have been described in Sambrook *et al*. (2nd ed.), Cold Spring Harbor Laboratory, Cold Spring Harbor (1982, 1989); *Methods in Enzymol*. (Vols. 68, 100, 101, 118, and 152-155) (1979, 1983, 1986 and 1987); and *DNA Cloning,* D.M. Clover, Ed., IRL Press, Oxford (1985).

Assembly method using oligonucleotide directed double-strand break repair is particularly flexible where it does not require the presence of any restriction sites within the target DNA sequence. The cost of this method is low because of the reduction in the total length of synthetic oligonucleotide needed to construct the target long DNA sequence. Only one DNA strand of the target DNA sequence needs to be obtained synthetically, compared to conventional methods where both DNA strands are made synthetically. The method is accurate with low frequency of sequence error, because the *in vivo* double-strand repair rather than the *in vitro* ligation allows for a biological selection of the unmodified oligonucleotides and the subsequent screening of insert containing colonies further eliminates the undesired recombination products.

### 2. Assembly using PCR-based methods.

PCR-based methods may also be employed to assemble short oligonucleotides into long DNA sequences. *PCR Technology: Principles and Applications for DNA Amplification* (ed. H.A. Erlich, Freeman Press, NY, NY, 1992. The polymerase used to direct the nucleotide synthesis may include, for example, *E. coli* DNA polymerase I, Klenow fragment of *E. coli* DNA polymerase, polymerase muteins, heat-stable enzymes, such as Taq polymerase, Vent polymerase, and the like.

As an example, self-priming PCR or DNA shuffling methods may also be used for assembling short overlapping oligonucleotides into long DNA sequences. Dillon *et al., BioTechniques* 9(3):298-300 (1990), Hayashi *et al., BioTechniques* 17(2):310-315 (1994), Chen *et al., J. Am. Chem. Soc*. 116:8799-8800 (1994), Prodromou *et al., Protein Engineering* 5(8):827-829 (1992), Stemmer *et al., Gene* 164:49-53 (1995), U.S. Patent Nos. 5,830,721, 5,811,238, 5,830,721, 5,605,793, 5,834,252, and 5,837,458, and PCT publications WO 98/13487, WO98/27230, WO 98/31837, WO 99/41402, 99/57128, and WO 99/65927. Essentially, overlapping oligonucleotides, which collectively represent the target long DNA sequence, are mixed and subjected to PCR reactions, such that those overlapping at their 3' ends are extended to give longer double-strand products and repeated until the full-sized target sequence is obtained.

The overlapping oligonucleotides may be mixed in a standard PCR containing dNTP, DNA polymerase of choice, and buffer. The overlapping ends of the oligonucleotides, upon annealing, create short regions of double-strand DNA and serve as primers for the elongation by DNA polymerase in a PCR reaction. Products of the elongation reaction serve as substrates for formation of a longer double-strand DNA, eventually resulting in the synthesis of full-length target sequence. The PCR conditions may be optimized to increase the yield of the target long DNA sequence. The choice of the DNA polymerase for the PCR reactions is based on its properties. For example, thermostable polymerases, such as Taq polymerase may be used. In addition, Vent DNA polymerase may be chosen in preference to Taq polymerase because it possesses a 3'-5' proofreading activity, a strand displacement activity and a much lower terminal transferase activity, all of which serve to improve the efficiency and fidelity of the PCR reactions.

Although it is possible to obtain the target sequence in a single step by mixing all the overlapping oligonucleotides, PCR reactions may also be performed in multiple steps, such that larger sequences might be assembled from a series of separate PCR reactions whose products are mixed and subjected to a second round of PCR. For example, it has been shown that the addition of 5' and 3' primers at the end of first round PCR reactions may be advantageous to generate the full-length DNA product. In other instances, additional sequences, such as restriction sites, a Shine-Dalgarno sequence, and a transcription terminator, among other, may be desirably added to the target sequence to facilitate the subsequent cloning of the target sequence gene into expression vectors. These new sequences may require additional primers and additional PCR reactions. Moreover, if the self-priming PCR fails to give a full-sized product from a single reaction, the assembly may be rescued by separately PCR-amplifying pairs of overlapping oligonucleotides, or smaller sections of the target DNA sequence, or by the conventional filling-in and ligation method.

Successful assembly of the target long DNA sequence may be verified by DNA sequencing, hybridization-based diagnostic method, molecular biology techniques, such as restriction digest, selection marker, or other suitable methods. DNA manipulations and enzyme treatments are carried out in accordance with established protocols in the art and manufacturers' recommended procedures. Suitable techniques have been described in Sambrook *et al*. (2nd ed.), Cold Spring Harbor Laboratory, Cold Spring Harbor (1982, 1989); *Methods in Enzymol.* (Vols. 68, 100, 101, 118, and 152-155) (1979, 1983, 1986 and 1987); and *DNA Cloning,* D.M. Clover, Ed., IRL Press, Oxford (1985).

There are several advantages to the assembly method using PCR-based methods. It generally requires neither phosphorylation nor ligation, while giving high yields. The cost of this method is relatively low because it reduces the number of oligonucleotides needed for synthetic constructions. Only oligonucleotides representing the partial sequence of each strand are synthesized and the gaps in the annealed oligonucleotides are filled in using DNA polymerase during PCR. The assembly of overlapping oligonucleotides may be achieved in a one-pot single step PCR with no requirement for isolation and purification of intermediate products. In particular, gel purification of oligonucleotides are not necessary and crude oligonucleotide preparations may be directly used for PCR. Furthermore, this method of assembly is accurate and does not require the existence of restriction enzyme sites in the target sequence.

### EXAMPLES

The following examples further illustrate the present invention. These examples are intended merely to be illustrative of the present invention and are not to be construed as being limiting. The examples are intended specifically to illustrate which may be attained using the process within the scope of the present invention.

### EXAMPLE 1

### Preparation of solid supports

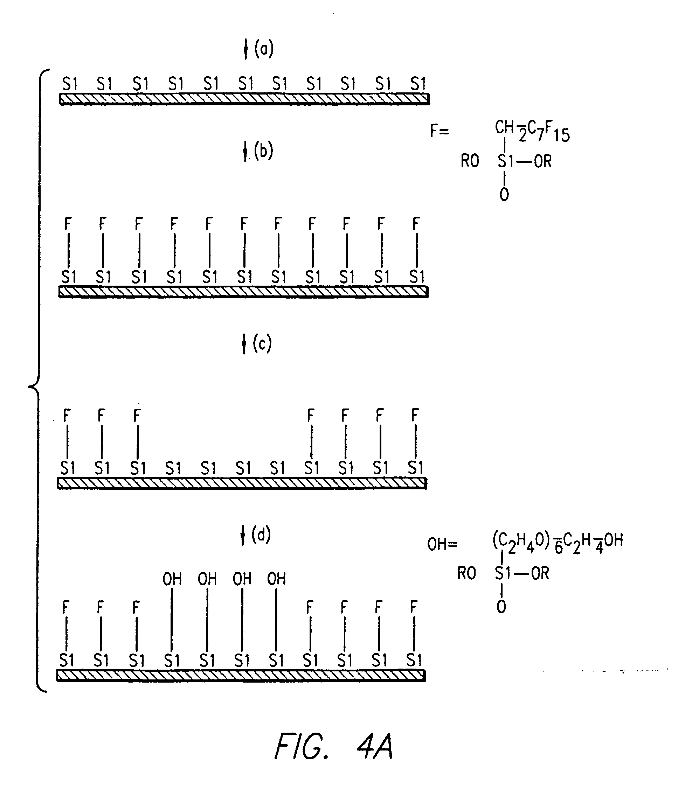

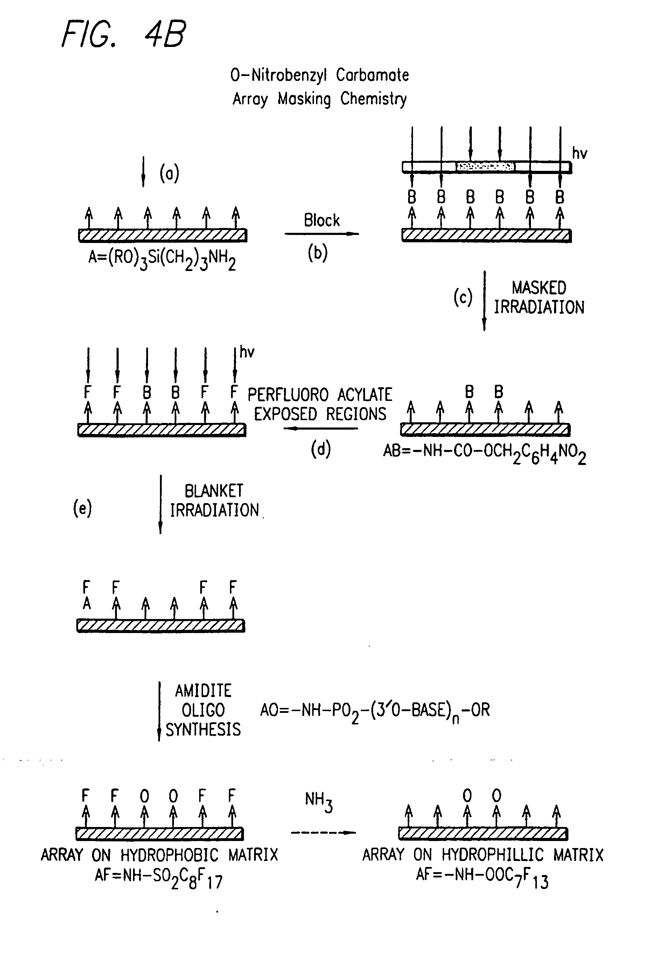

The oligonucleotides are synthesized on a glass plate. The plate is first coated with the stable fluorosiloxane 3-(1,1-dihydroperfluoroctyloxy) propyltriethoxysilane. A CO₂ laser is used to ablate off regions of the fluorosiloxane and expose the underlying silicon dioxide glass. The plate is then coated with glycidyloxypropyl trimethoxysilane, which reacts only on the exposed regions of the glass to form a glycidyl epoxide. The plate is next treated with hexaethyleneglycol and sulfuric acid to convert the glycidyl epoxide into a hydroxyalkyl group, which acts as a linker arm. The hydroxyalkyl group resembles the 5'-hydroxide of nucleotides and provides a stable anchor on which to initiate solid phase synthesis. The hydroxyalkyl linker arm provides an average distance of 3-4 nm between the oligonucleotide and the glass surface. The siloxane linkage to the glass is completely stable to all acidic and basic deblocking conditions typically used in oligonucleotide synthesis. This scheme for preparing array plates is illustrated in Figures 4A and 4B.

### EXAMPLE 2

### Oligonucleotide synthesis on a solid support

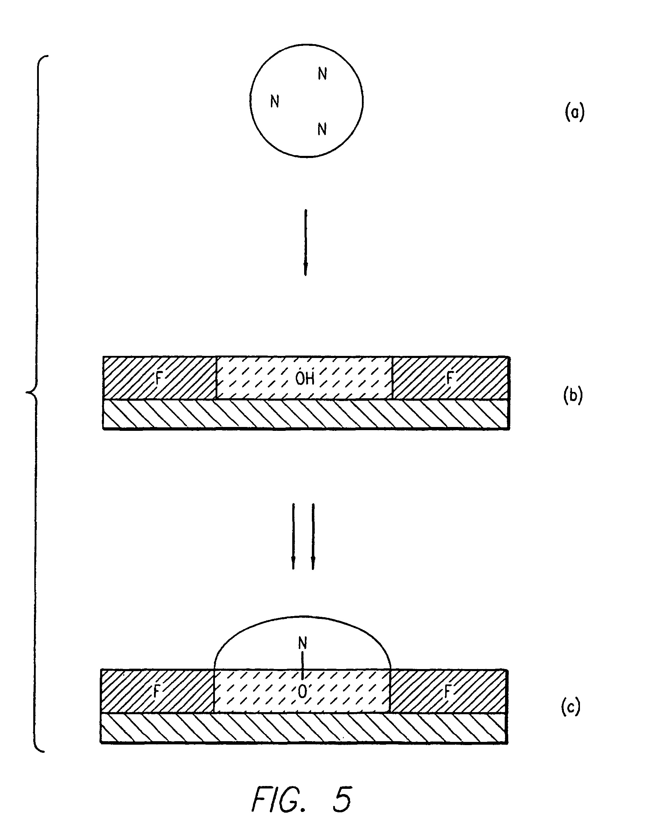

The hydroxyalkylsiloxane surface in the dots has a surface tension of approximately γ=47, whereas the fluoroxysilane has a surface tension of γ=18. For oligonucleotide assembly, the solvents of choice are acetonitrile, which has a surface tension of γ =29, and diethylglycol dimethyl ether. The hydroxyalkylsiloxane surface is thus completely wet by acetonitrile, while the fluorosiloxane masked surface between the dots is very poorly wet by acetonitrile. Droplets of oligonucleotide synthesis reagents in acetonitrile are applied to the dot surfaces and tend to bead up, as shown in Figure 5. Mixing between adjacent dots is prevented by the very hydrophobic barrier of the mask. The contact angle for acetonitrile at the mask-dot interface is approximately θ=43°. The plate effectively acts as an array microliter dish, wherein the individual wells are defined by surface tension rather than gravity. The volume of a 40 micron droplet is 33 picoliter. The maximum volume retained by a 50 micron dot is approximately 100 picoliter, or about 3 droplets. A 100 micron dot retains approximately 400 picoliter, or about 12 droplets. At maximum loading, 50 micron and 100 micron dots bind about 0.07 and 0.27 femtomoles oligonucleotide, respectively.

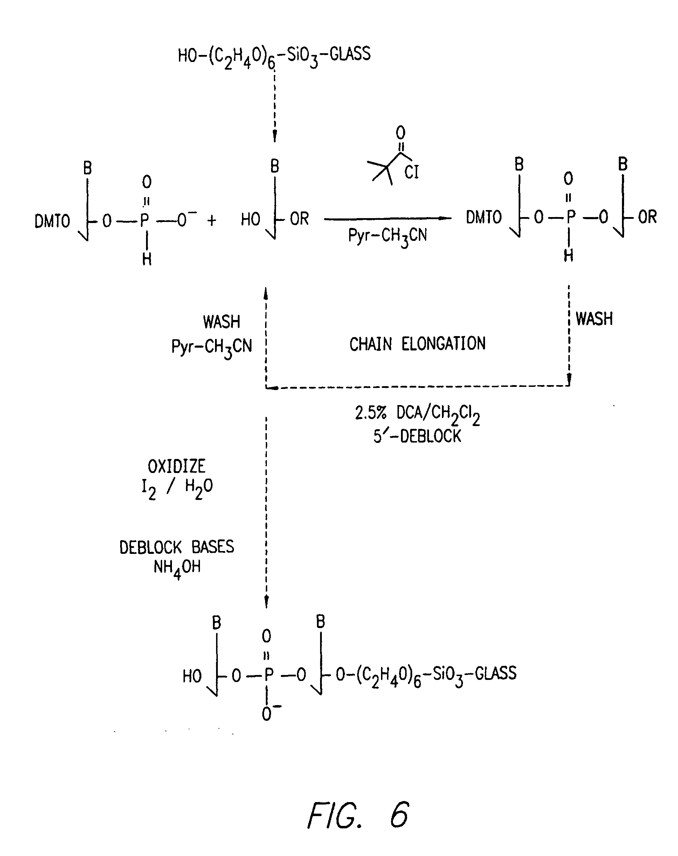

Oligonucleotide synthesis on the prepared dots (Figure 4B, bottom) is carried out according to the H-phosphonate procedure (Figure 6), or by the phosphoroamidite method. Both methods are well known to those of ordinary skill in the art. Christodoulou, C., "Oligonucleotide Synthesis" in "Protocols for oligonucleotides and analogs; synthesis and properties," *Methods Mol. Biol.* 20:19-31 (1993) and Beaucage, S., "Oligodeoxyribonucleotides Synthesis" in "Protocols for oligonucleotides and analogs; synthesis and properties," *Methods Mol. Biol*. 20:33-61 (1993). Delivery of the appropriate blocked nucleotides and activating agents in acetonitrile is directed to individual dots using the picopump apparatus described in Example 3. All other steps, (e.g., DMT deblocking, washing) are performed on the array in a batch process by flooding the surface with the appropriate reagents. An eight nozzle piezoelectric pump head is used to deliver the blocked nucleotides and activating reagents to the individual dots, and delivering droplets at 1000 Hz, requires only 32 seconds to lay down a 512x512 (262k) array. Since none of the coupling steps have critical time requirements, the difference in reaction time between the first and lost droplet applied is insignificant.

### EXAMPLE 3

### Construction of Piezoelectric Impulse Jet Pump Apparatus

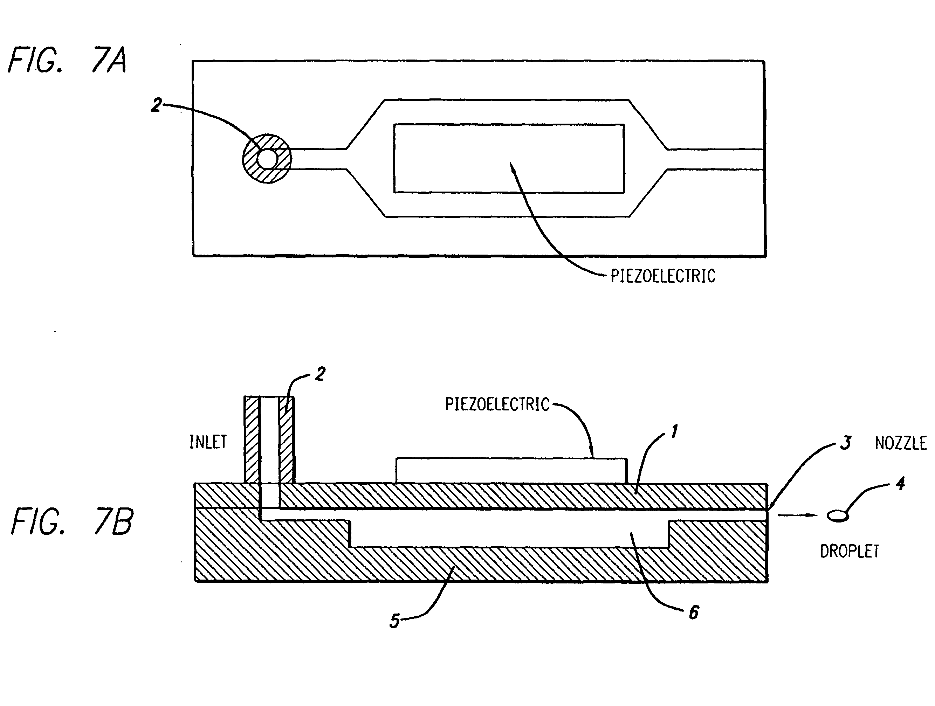

Piezoelectric impulse jets are fabricated from Photoceram (Coming Glass, Coming, N.Y.), a UV sensitive ceramic, using standard photolithographic techniques to produce the pump details. The ceramic is fired to convert it to a glassy state. The resulting blank is then etched by hydrogen fluoride, which acts faster in exposed then in nonexposed areas. After the cavity and nozzle details are lapped to the appropriate thickness in one plate, the completed chamber is formed by diffusion bonding a second (top) plate to the first plate. The nozzle face is lapped flat and surface treated, then the piezoelectric element is epoxied to the outside of the pumping chamber. When the piezoelectric element is energized it deforms the cavity much like a one-sided bellows, as shown in Figure 7.

To determine the appropriate orifice size for accurate firing of acetonitrile droplets, a jet head with a series of decreasing orifice sizes is prepared and tested. A 40 micron nozzle produces droplets of about 65 picoliter.

A separate nozzle array head is provided for each of the four nucleotides and a fifth head is provided to deliver the activating reagent for coupling. The five heads are stacked together with a mechanically defined spacing. Each head has an array of eight nozzles with a separation of 400 microns.

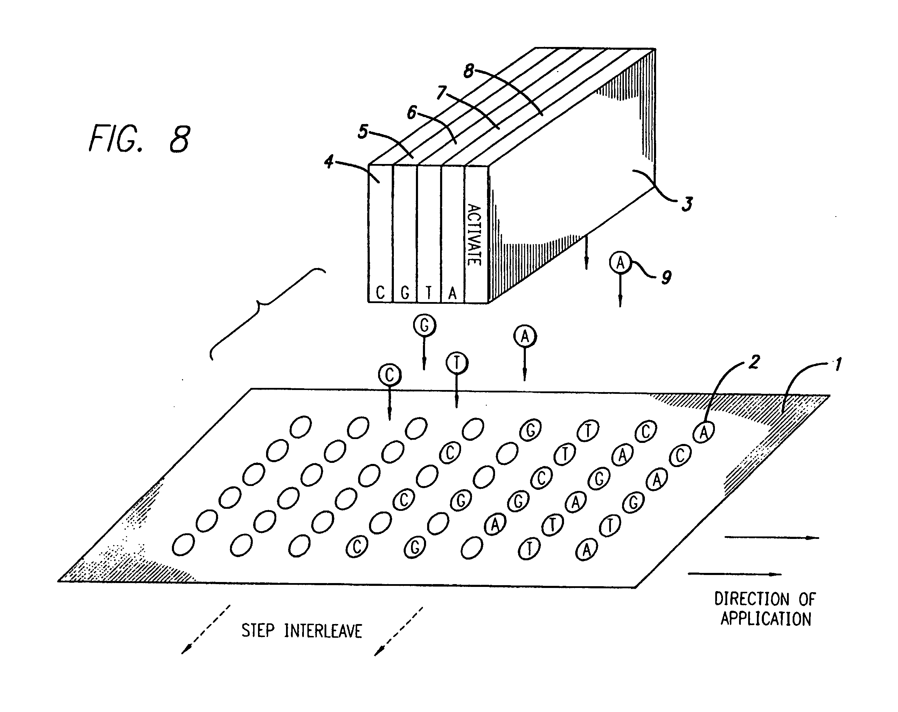

The completed pump unit is assembled with the heads held stationary and the droplets fired downward at a moving array plate as shown in Figure 8. The completed pump unit assembly (3) consists of nozzle array heads (4-7) for each of the four nucleotidase and a fifth head (8) for activating reagent. When energized, a microdroplet (9) is ejected from the pump nozzle and deposited on the array plate (1) at a functionalized binding site (2).

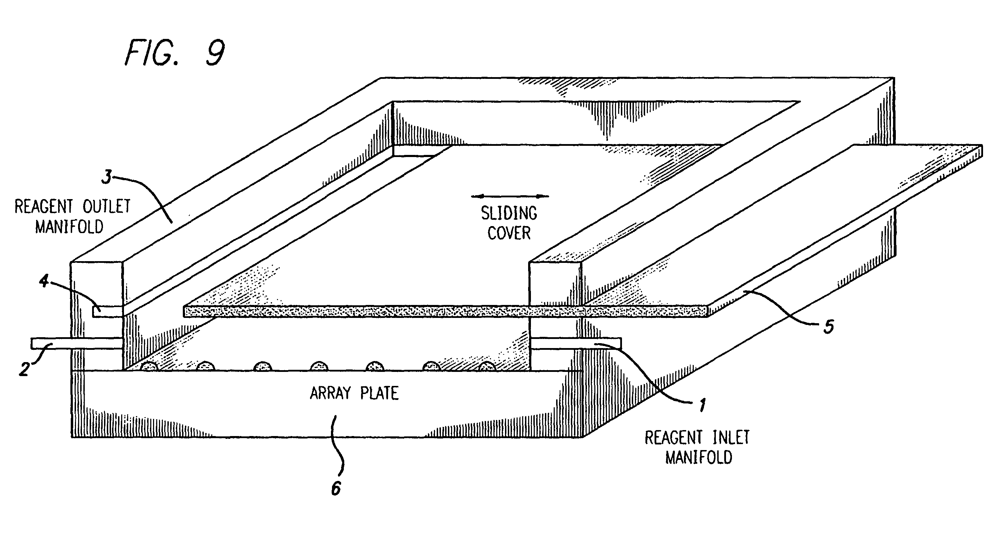

A plate holding the target array is held in a mechanical stage and is indexed in the X and Y planes beneath the heads by a synchronous screw drives: The mechanical stage is similar to those used in small milling machines, microscopes and microtomes, and provides reproducible positioning accuracy better than 2.5 microns or 0.1 mil. As shown in Figure 9, the plate holder (3) is fitted with a slotted spacer (4) which permits a cover plate (5) to be slid over the array (6) to form an enclosed chamber. Peripheral inlet (1) and outlet (2) ports are provided to allow the plate to be flooded for washing, application of reagents for a common array reaction, or blowing the plate dry for the next dot array application cycle.

Both the stage and head assembly are enclosed in a glove box which can be evacuated or purged with argon to maintain anhydrous conditions. With the plate holder slid out of the way, the inlet lines to the heads can be pressurized for positive displacement priming of the head chambers or flushing with clean solvent. During operation, the reagent vials are maintained at the ambient pressure of the box.

With a six minute chemistry cycle time, the apparatus can produce 10-mer array plates at the rate of 1 plate or 10⁶ oligonucleotides per hour.

### EXAMPLE 4

### In situ synthesis of cleavable oligonucleotides

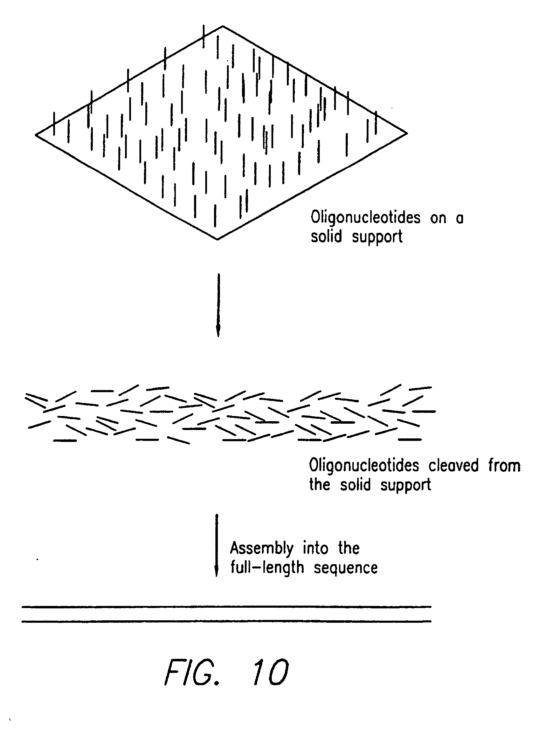

Surface tension array synthesis is a two step process, substrate surface preparation followed by *in situ* oligonucleotide synthesis. Substrate preparation begins with glass cleaning in detergent, then base and acid (2% Micro 90, 10% NaOH and 10% H₂SO₄) followed by spin coating with a layer of Microposit 1818 photoresist (Shipley, Marlboro, MA) that is soft baking at 90°C for 30 min. The photoresist is then patterned with UV light at 60 m Watts/cm² using a mask that defines the desired size and distribution of the array features. The exposed photoresist is developed by immersion in Microposit 351 Developer (Shipley, Marlboro, MA) followed by curing at 120°C for 20 minutes. Substrates are then immersed in 1% solution of tridecafluoro-1,1,2,2-tetrahydrooctyl)-1-trichlorosilane (United Chemical Technology, Bristol, PA) in dry toluene to generate a hydrophobic silane layer surrounding the array features which are still protected by photoresist. The fluorosilane is cured at 90°C for 30 min then treated with acetone to remove the remaining photoresist. The exposed feature sites are coated with 1% 4-aminobutyldimethylmethoxysilane (United Chemical Technology, Bristol, PA) then cured for 30 min at 105°C. Finally these sites are coupled with TOPS amidites that will support subsequent oligonucleotide synthesis. A schematic diagram of long DNA sequence assembly is shown in Figure 10.

Surface tension patterned substrates are aligned on a chuck mounted on the X-Y stage of a robotic array synthesizer where piezoelectric nozzles (Microfab Technologies, Plano, TX) are used to deliver solutions of activated standard H-phosphonate amidites (Froehler *et al., Nucleic Acids Res.* 14:5339-5407 (1986)). The piezoelectric jets are run at 6.67 kHz using a two-step waveform, which fires individual droplets of approximately 50 picoliters. Washing, deblocking, capping, and oxidizing reagents are delivered by bulk flooding the reagent onto the substrate surface and spinning the chuck mount to remove excess reagents between reactions. The substrate surface is environmentally protected throughout the synthesis by a blanket of dry N₂ gas. Localizing and metering amidite delivery is mediated by a computer command file that directs delivery of the four amidites during each pass of the piezoelectric nozzle bank so a predetermined oligonucleotide is synthesized at each array coordinate.

Piezoelectric printed oligonucleotide synthesis is performed using the following reagents (Glen Research, Sterling, VA): phosphoramidites: pac-dA-CE phosphoramidite, Ac-dC-CE phosphoramidite, iPr-pac-dG-CE phosphoramidite, dT CE phosphoramidite (all at 0.1M); activator: 5-ethylthio tetrazole (0.45M). Amidites and activator solutions are premixed, 1:1:v/v, in a 90% adiponitrile (Aldrich, Milwaukee, WI): 10% acetonitrile solution prior to synthesis. Ancillary reagents are oxidizer (0.1M iodine in THF/pyridine/water), Cap mix A (THF/2,6-lutidine/acetic anhydride), Cap mix B (10% 1-methylimidazole/THF), and 3% TCA in DCM.

## Claims

1. A method for producing a DNA sequence of greater than 200 bases long comprising the steps of:
(a) synthesizing on a functionalized solid support at least ten oligonucleotides from about 10 to 200 bases encoding for either the sense or antisense strand of said DNA sequence wherein said oligonucleotides are covalently attached to the solid support using a cleavable moiety;
(b) cleaving said oligonucleotides from the solid support; and
(c) assembling the oligonucleotides into said DNA sequence; wherein the functionalized solid support comprises patterned regions of hydrophilic and hydrophobic surfaces, the functionalized solid support comprising functionalized sites each 50-2000 microns in diameter.

2. The method according to claim 1 wherein said functionalized solid support is a functionalized glass plate, a functionalized silicon or a functionalized wafer.

3. The method according to claim 1 wherein said functionalized solid support is selected from the group consisting of functionalized polystyrene, functionalized polyethylene, functionalized polypropylene, and functionalized polytetrafluorethylene.

4. The method according to any of claims 1 to 3, wherein said cleavage is enzymatic cleavage, photolytic cleavage, chemical cleavage or thermal cleavage.

5. The method according to any of claims 1 to 4, wherein said synthesis further comprises coupling of a phosphoramidite to a reactive group on said functionlaized solid support to convert said reactive group into a cleavable linker.

6. The method according to any of claims 1 to 5, wherein said functionalized solid support comprises 10 x 10⁻¹⁵ to 50 x 10⁻¹⁵ moles of functionalized binding sites per mm².

7. The method according to any of claims 1 to 6, wherein said synthesis is performed using a piezoelectric pump.

8. The method according to any of claims 1 to 7, wherein step (c) further comprises enzymatic ligation.

9. The method according to any of claims 1 to 8, wherein step (c) further comprises the use of a DNA polymerase.

10. The method according to any of claims 1 to 9, wherein said DNA sequence encodes a gene, a plasmid or a virus.

11. The method according to any of claims 1 to 10, wherein said DNA sequence is the genome of an organism.

12. A solid support containing a cleavable moiety for oligonucleotide synthesis as defined in any one of claims 1 to 3 or claim 6.

## Patentansprüche

1. Verfahren zur Herstellung einer DNA-Sequenz mit mehr als 200 Basen Länge, umfassend die folgenden Schritte:
(a) Synthetisieren von mindestens zehn Oligonukleotiden aus etwa 10 bis 200 Basen, die entweder für den Sense- oder Antisense-Strang der DNA-Sequenz kodieren, auf einem funktionalisierten festen Träger, wobei die Oligonukleotide kovalent mit einer abspaltbaren Komponente an den festen Träger gebunden sind;
(b) Abspalten der Oligonukleotide vom festen Träger; und
(c) Zusammensetzen der Oligonukleotide zur DNA-Sequenz;
wobei der funktionalisierte feste Träger gemusterte Bereiche von hydrophilen und hydrophoben Flächen umfasst, wobei der funktionalisierte feste Träger funktionalisierte Stellen umfasst, die jeweils 50 - 2000 µm Durchmesser haben.

2. Verfahren nach Anspruch 1, wobei der funktionalisierte feste Träger eine funktionalisierte Glasplatte, ein funktionalisiertes Silizium oder ein funktionalisierter Wafer ist.

3. Verfahren nach Anspruch 1, wobei der funktionalisierte feste Träger aus der Gruppe bestehend aus funktionalisiertem Polystyrol, funktionalisiertem Polyethylen, funktionalisiertem Polypropylen und funktionalisiertem Polytetrafluorethylen ausgewählt ist.

4. Verfahren nach einem der Ansprüche 1 bis 3, wobei die Abspaltung eine enzymatische Abspaltung, photolytische Abspaltung, chemische Abspaltung oder thermische Abspaltung ist.

5. Verfahren nach einem der Ansprüche 1 bis 4, wobei die Synthese weiter das Koppeln eines Phosphoramidits mit einer reaktiven Gruppe auf dem funktionalisierten festen Träger umfasst, um die reaktive Gruppe in einen abspaltbaren Linker umzuwandeln.

6. Verfahren nach einem der Ansprüche 1 bis 5, wobei der funktionalisierte feste Träger 10 x 10⁻¹⁵ bis 50 x 10⁻¹⁵ mol funktionalisierte Bindungsstellen pro mm² umfasst.

7. Verfahren nach einem der Ansprüche 1 bis 6, wobei die Synthese mit einer piezoelektrischen Pumpe durchgeführt wird.

8. Verfahren nach einem der Ansprüche 1 bis 7, wobei Schritt (c) weiter die enzymatische Ligation umfasst.

9. Verfahren nach einem der Ansprüche 1 bis 8, wobei Schritt (c) weiter die Verwendung einer DNA-Polymerase umfasst.

10. Verfahren nach einem der Ansprüche 1 bis 9, wobei die DNA-Sequenz für ein Gen, ein Plasmid oder ein Virus kodiert.

11. Verfahren nach einem der Ansprüche 1 bis 10, wobei die DNA-Sequenz das Genom eines Organismus ist.

12. Fester Träger, enthaltend eine abspaltbare Komponente für die Oligonukleotidsynthese, wie in einem der Ansprüche 1 bis 3 oder Anspruch 6 definiert.

## Revendications

1. Un procédé pour synthétiser une séquence ADN de plus de 200 bases comprenant les étapes suivantes :
(a) La synthèse sur support solide fonctionnalisé d'au moins dix oligonucléotides de 10 à 200 bases encodant soit le brin sens soit le brin antisens de ladite séquence ADN, **caractérisée en ce que** lesdits oligonucléotides sont liés au support solide de manière covalente par une partie clivable ;
(b) Le clivage desdits oligonucléotides du support solide ; et
(c) L'assemblage des oligonucléotides en ladite séquence ADN, **caractérisé en ce que** le support solide fonctionnalisé comprend un motif de régions de surfaces hydrophiles et hydrophobes, le support solide fonctionnalisé comportant des sites fonctionnalisés de 50-2000 microns de diamètre chacun.

2. Le procédé selon la revendication 1 **caractérisé en ce que** ledit support solide fonctionnalisé est une plaque de verre fonctionnalisée, une surface de silicone fonctionnalisé ou un wafer fonctionnalisé.

3. Le procédé selon la revendication 1 **caractérisé en ce que** ledit support solide fonctionnalisé est choisi parmi le groupe que forment le polystyrène fonctionnalisé, le polyéthylène fonctionnalisé, le polypropylène fonctionnalisé, et le polytétrafluoroéthylène fonctionnalisé.

4. Le procédé selon l'une quelconque des revendications 1 à 3 **caractérisé en ce que** ledit clivage est un clivage enzymatique, un clivage photolytique, un clivage chimique ou un clivage thermique.

5. Le procédé selon l'une quelconque des revendications 1 à 4 **caractérisé en ce que** ladite synthèse comprend également le couplage d'un phosphoramidité avec un groupe réactif sur ledit support solide fonctionnalisé afin de convertir ledit groupe réactif en un liant clivable.

6. Le procédé selon l'une quelconque des revendications 1 à 5 **caractérisé en ce que** ledit support solide fonctionnalisé comporte 10 x 10⁻¹⁵ à 50 x 10⁻¹⁵ moles de sites de liaison fonctionnalisés par mm².

7. Le procédé selon l'une quelconque des revendications 1 à 6 **caractérisé en ce que** la dite synthèse est effectuée à l'aide d'une pompe piézoélectrique.

8. Le procédé selon l'une quelconque des revendications 1 à 7 **caractérisé en ce que** l'étape (c) comprend également une ligation enzymatique.

9. Le procédé selon l'une quelconque des revendications 1 à 8 **caractérisé en ce que** l'étape (c) fait également appel à une ADN polymérase.

10. Le procédé selon l'une quelconque des revendications 1 à 9 **caractérisé en ce que** ladite séquence d'ADN encode un gène, un plasmide ou un virus.

11. Le procédé selon l'une quelconque des revendications 1 à 10 **caractérisé en ce que** ladite séquence d'ADN est le génome d'un organisme.

12. Un support solide comportant une partie clivable pour la synthèse d'oligonucléotides telle que définie selon l'une quelconque des revendications 1 à 3 ou la revendication 6.
